**Europäisches Patentamt**

**European Patent Office**

**Office européen des brevets**

(19)

(11) Veröffentlichungsnummer : **0 393 072 B1**

(12)

# EUROPÄISCHE PATENTSCHRIFT

(45) Veröffentlichungstag der Patentschrift :
20.01.93 Patentblatt 93/03

(51) Int. Cl.$^5$ : **A61K 7/50**, C11D 3/37

(21) Anmeldenummer : **88909314.2**

(22) Anmeldetag : **26.10.88**

(86) Internationale Anmeldenummer :
**PCT/EP88/00963**

(87) Internationale Veröffentlichungsnummer :
**WO 89/03669 05.05.89 Gazette 89/10**

(54) **WASSERFREIE HAUTREINIGUNGSMITTEL UND IHRE VERWENDUNG.**

(30) Priorität : **30.10.87 DE 3736970**

(43) Veröffentlichungstag der Anmeldung :
**24.10.90 Patentblatt 90/43**

(45) Bekanntmachung des Hinweises auf die
Patenterteilung :
**20.01.93 Patentblatt 93/03**

(84) Benannte Vertragsstaaten :
**AT BE CH DE FR GB IT LI LU NL SE**

(56) Entgegenhaltungen :
**DE-A- 1 949 849
DE-A- 3 517 385
Patent Abstracts of Japan, Band 8, Nr. 30,
C209, Zusammenfassung von JP 58-192814,
publ. 1983.11.10**

(56) Entgegenhaltungen :
**Patent Abstracts of Japan, Band 9, Nr. 317,
C319, Zusammenfasung von JP 60-152407,
publ. 1985.08.10
Patent Abstracts of Japan, Band 10, Nr. 377,
C392, Zusammenfassung von JP 61-171415,
publ. 1086.08.02**

(73) Patentinhaber : **Chemische Fabrik
Stockhausen & Cie.
Bäkerpfad 25
W-4150 Krefeld (DE)**

(72) Erfinder : **BOUILLON, Günter
Tulpenstrasse 31
W-4152 Kempen (DE)**

(74) Vertreter : **Klöpsch, Gerald, Dr.-Ing.
Patentanwalt
An Gross St. Martin 6
W-5000 Köln 1 (DE)**

## Beschreibung

Die Erfindung betrifft ein Hautreinigungsmittel, vorzugsweise in fließfähiger oder pastöser Form, das wasserunlösliche, jedoch wasserquellbare Feststoffteilchen von organischen Polymeren natürlichen oder synthetischen Ursprungs enthält.

Hautreinigungsmittel in fließfähiger oder pastöser Form werden allgemein zur Reinigung stark verschmutzter Hautpartien verwendet, z.B. in der metallbearbeitenden Industrie und Lackindustrie. Bekannte derartige Hautreinigungsformulierungen, meistens Pasten, enthalten Tenside als waschaktive Substanz, oleophile und/oder oleophobe Lösevermittler, organische Lösemittel, sowie gegebenenfalls Wasser. Diese bekannten Produkte enthalten ferner als Reibemittel Sand, Holzmehl oder Kunststoffpulver. Diese Feststoffe wirken wegen ihrer Abrasivität als Schmutzentferner. Sie verhindern allerdings nicht, daß der einmal gelöste oder emulgierte Schmutz bei anschließendem Verdünnen mit Wasser koaguliert und sich zumindest teilweise wieder auf der Haut ablagert.

Aufgabe der Erfindung ist es, die bekannten Hautreinigungsmittel in ihrer Reinigungswirkung zu verbessern, insbesondere eine Koagulierung und Wiederablagerung des gelösten bzw. emulgierten Schmutzes zu verhindern.

Diese Aufgabe wird bei einem Hautreinigungsmittel, welches Tenside, organische Lösungsmittel, wasserunlösliche Reibmittel sowie gegebenenfalls Verdickungsmittel, Gerüststoffe, Parfümstoffe, Konservierungsstoffe, Farbstoffe, Antioxidantien und/oder keratolytische Wirkstoffe enthält, durch einen Zusatz an wasserunlöslichen, in Wasser lediglich quellbaren organischen Polymeren gelöst.

Gegenstand der Erfindung ist somit ein wasserfreies Hautreinigungsmittel enthaltend Tenside, organische Lösungsmittel und wasserunlösliche Reibmittel sowie gegebenenfalls Verdickungsmittel, Gerüststoffe, Parfümstoffe, Konservierungsstoffe, Farbstoffe, Antioxidantien und/oder keratolytische Wirkstoffe, das gekennzeichnet ist durch einen Gehalt an wasserunlöslichen, jedoch wasserquellbaren organischen Polymeren.

Die wasserunlöslichen, jedoch wasserquellbaren Polymeren liegen als Feststoffteilchen vor und wirken als Schmutzträger derart, daß der gelöste bzw. emulgierte Schmutz bei anschließendem Verdünnen mit Wasser nicht wieder koaguliert und wieder abgelagert, sondern abtransportiert wird. Darüberhinaus kann das wasserunlösliche, wasserquellbare Polymer in der wasserfreien Formulierung als Reibmittel wirken und entweder als alleiniges Reibmittel oder zusammen mit weiteren, an sich bekannten Reibemitteln eingesetzt werden. In der wasserfreien Formulierung umhüllt der Schmutz die Partikel des Polymeren, die beim anschließenden Anfeuchten der Hände unter Wasseraufnahme stark anquellen und die Koagulierung des Schmutzes und damit das Wiederanschmutzen der Haut verhindern. Hierzu ist die Wasserunlöslichkeit der Partikel unbedingte Voraussetzung. Die Feststoffteilchen des Polymeren können darüberhinaus in der wasserfreien Formulierung, falls sie nicht zu feinteilig sind, auch als Reibemittel wirken, indem sie durch ihre Abrasivität die Verschmutzung mechanisch abtragen.

Die erfindungsgemäße Wirkung der wasserquellbaren Polymerpartikel ist sowohl neu als auch überraschend: In der DE-A 35 17 385 wird zwar ein kosmetisches Hautreinigungsmittel beschrieben, das jedoch in einer wasserfreien Fettphase einen Emulgator und ein wasserlösliches Polymerisat als Reibmittel enthält. Die Wasserlöslichkeit des Polymeren ist zwingende Voraussetzung dafür, das Reibmittel wieder restlos durch Wasser von der Gesichtshaut entfernen zu können. Das Problem der Wiederablagerung von bereits abgelöstem Schmutz und damit das Wiederanschmutzen der Haut wird hier nicht angesprochen.

Als in Wasser unlösliche, vielmehr lediglich quellbare Feststoffe werden organische Polymere natürlichen oder synthetischen Ursprungs eingesetzt. Es kann sich dabei um Polymerisate auf Basis von modifizierten Naturstoffen oder auf Basis von synthetischen Produkten handeln. Die Wasserunlöslichkeit wird im allgemeinen durch Vernetzung erzielt. Der Begriff Polymerisate umfasst sowohl die Homo- als auch die Co- und Terpolymeren.

Geeignete organische Polymere auf Basis modifizierter Naturstoffe sind beispielsweise die Produkte auf Stärke- und Zellulosebasis, die durch Pfropfung mit vozugsweise Acrylderivaten modifiziert werden können. Als Acrylderivate sind zu nennen (Meth-)acryl säure und deren Salze, (Meth-)acrylnitril, (Meth-)acrylamid und die (Meth-)acrylester, sowie die partiellen Verseifungsprodukte dieser Acrylderivate.

Als synthetische organische Polymere sind die Homo- und Copolymerisate, insbesondere der vorstehend genannten Acrylderivate geeignet, wobei insbesondere die Copolymerisate untereinander oder mit Isobutylen und Maleinsäureanhydrid zu nennen sind. Weiter sind auch Polyurethane geeignet. Als Comonomeren können die Polymerisate enthalten Acrylamidopropansulfonsäure, Vinylphosphonsäure, Vinylsulfonsäure, Dialkylaminoalkyl(meth-)acrylate, Dialkylamino(meth-)acrylamide sowie die quaternierten Formen der beiden vorstehend genannten basischen Comonomeren.

Neben den erfindungsgemäßen wasserquellbaren Feststoffpartikeln können bekannte nicht quellbare Reibemittel wie Holzmehl, Kunststoffpulver oder Sand verwendet werden.

2

Als waschaktive Substanz (Tenside) oder Emulgatoren enthalten die erfindungsgemäßen Hautreinigungsmittel bevorzugt alkoxylierte Fettalkohole, alkoxylierte Fettsäuren, alkoxylierte Fettsäureglyceride, alkoxylierte Alkylphenole oder Alkylsulfate, Alkylethersulfate, Sorbitan-Fettsäureester, Fettsäureglyceride, ohne daß hier eine Beschränkung auf die aufgezählten Produkte beabsichtigt ist.

Als Lösemittel werden in dem erfindungsgemäßen Hautreinigungsmittel Carbonsäureester,ferner paraffinische oder isoparaffinische Kohlenwasserstoffe, Arylcarbonsäureester oder Terpene allein oder in Mischung untereinander eingesetzt.

Als Carbonsäureester werden die Ester von Alkoholen mit ein- oder mehrbasischen Carbonsäuren eingesetzt, die hierfür verwendeten Alkohole sind gerad- oder verzweigtkettig und haben bevorzugt 1 bis 8 Kohlenstoffatome, wobei die niederen Alkohole mit 1 bis 4 C-Atome bevorzugt sind. Beispiele sind Methanol, Ethanol, n-Propanol, Isopropanol, n-Butanol und Isobutanol. Als Beispiel für einen höheren Alkohol ist Isooctanol zu nennen. Die Carbonsäure der Carbonsäureester weist bevorzugt 1 bis 10 Kohlenstoffatome auf und kann ebenfalls gerad- oder verzweigtkettig sein. Beispiele für Carbonsäuren sind Ameisensäure, Essigsäure, Propionsäure für einbasische sowie Adipinsäure, Sebacinsäure oder Zitronensäure für mehrbasische Säuren. Beispiele für geeignete Carbonsäureester sind die als Lösemittel bekannten niedrigen Carbonsäureester, wie z.B. Butylacetat oder höher siedende Derivate, z.B. Methoxybutylacetat.

Als Verdickungsmittel werden Wachse, Zellulosederivate (wie Zelluloseether), synthetische Polymere, pyrogene Kieselsäure oder modifizierte Bentonite verwendet.

Die erfindungsgemäßen Hautreinigungsmittel können ferner Parfümstoffe, Konservierungsmittel, Rückfettungsmittel, Farbstoffe und/oder Antioxidantien enthalten.

Die Mengenverhältnisse der Komponenten können innerhalb weiter Grenzen schwanken. Der Gehalt an wasserunlöslichen, jedoch wasserquellbaren Polymerteilchen beträgt, bezogen auf das Gesamtgewicht aus waschaktiver Substanz (Tensid), Lösemittel und wasserquellbaren Polymer 0,3 bis 55, vorzugsweise 0,1 bis 17,6 insbesondere 1 bis 12,5 Gew.-%.

Bei gleicher Bezugsbasis wie für das wasserquellbare Polymer betragen die Gehalte an Lösemittel 5 bis 99, vorzugsweise 20 bis 98, insbesondere 30 bis 70 Gew.-% und für die waschaktive Substanz (Tensid) 0,7 bis 94,7 Gew.-%, vorzugsweise 1,4 bis 79,4 und insbesondere 29 bis 69 Gew.-%.

Die Menge der weiteren, gegebenenfalls noch vorhandenen Komponenten wie Verdickungsmittel, Rückfettungsmittel, Parfümstoffe, Konservierungsstoffe, Farbstoffe, Antioxidantien und keratolytische Wirkstoffe, die allein oder in Mischung vorhanden sein können, beträgt 0 bis 90 Gew.-%, bezogen auf die Gesamtrezeptur.

Die Teilchengröße der wasserquellbaren Feststoffpartikel ist kleiner als 1.000 μm, bevorzugt kleiner als 500 μm bei Verwendung als Schmutzträger und als Reibmittel; z.B. liegt die Korngröße im Bereich von etwa 50 bis 400 μm. Wird das wasserquellbare Polymer lediglich als Schmutzträger allein oder .zusammen mit anderen an sich bekannten Reibmitteln eingesetzt, so ist die Korngröße kleiner und liegt bei kleiner als 200 μm, bevorzugt bei kleiner als 150 μm, insbesondere kleiner als 100 μm, z.B im Bereich von 10 bis 200 μm. Entsprechende Kornfraktionen werden zweckmäßig durch Absieben erhalten.

Die Erfindung wird durch die nachfolgenden Beispiele erläutert:

Rezeptur I

15 Gew.-%    Ölsäure mit Triethanolamin neutralisiert (waschaktive Substanz)
58 Gew.-%    Butylacetat (Lösemittel)
5 Gew.-%    Sojaöl (Rückfetter)
5,1 Gew.-%    pyrogene Kieselsäure (Verdickungsmittel)
0,4 Gew.-%    Parfüm
9 Gew.-%    Polymerkomponente a (gemäß Tabelle I)
7,5 Gew.-%    Polyethylpulver, Korngröße kleiner als 300 μm

Rezeptur Ia

15 Gew.-%    Nonylphenol 9 EO
30 Gew.-%    iso-Hexadekan
55 Gew.-%    Polymerkomponente a (gemäß Tabelle I) kleiner als 100 μm

Um die Unterschiede in der Reinigungswirkung zwischen wasserlöslichen und nur wasserquellbaren Reibemitteln am Beispiel von Polymeren zu zeigen, wurde eine Standardrezeptur (Rezeptur II) entwickelt und in die nachfolgend beschriebenen Formulierungen jeweils gleiche Mengen an Polymerkomponenten (Tabelle I) gleicher Korngröße eingearbeitet.

Verwendete Rezeptur :

Rezeptur II:

        16,2 Gew.-% Fettalkohol 12/18 5 EO (waschaktive
                    Substanz)


    59,5 Gew.-% Estergemisch (Lösemittel)
     5,4 Gew.-% Rückfetter (Fettsäurealkylester)
    14,2 Gew.-% Verdickungsmittel (3,4 Gew.-% pyrogene
                    Kieselsäure; 7 Gew.-% Ethylenglykoldi-
                    stearat; 3,8 Gew.-% Celluloseaceto-
                    butyrat)
     0,4 Gew.-% Parfüm
     4,3 Gew.-% Polymerkomponenten a – f gemäß Tabelle I
    100   %


Tabelle I


Als Polymerkomponenten wurden verwendet:


        Handelsname :


a = Favor SAB 922   wasserquellbar vernetzte Polyacryl-
                                    säure teil-neutrali-
                                    siert


b = Rhodopol 23     wasserlöslich  Xanthangummi, Hetero-
                                    polysaccharid


c = Aqua-Keep       wasserquellbar wie a)


d = Praestol 2530   wasserlöslich  lineares Acrylamid-
                                    Natriumacrylat-Copolymer


e = Natrosol        wasserlöslich  Hydroxyalkylzellulose


f = Sunwet IM 1000 wasserquellbar Acrylsäure-modifizierte
                                    Stärke


Die so erhaltenen Formulierungen a bis f wurden nach dem folgenden Handwaschtest auf ihre Reinigungswirkung geprüft:

Methoden-Beschreibung

Handwaschtest

1. Material und Methode

1.1. Testmaterial

1.1.1. Lack (rot); schnelltrocknende, auf Fischölbasis aufgebauter Lack der Firma Rust Oleum.

1.2. Probanden

Das Testkollektiv besteht aus sechs hautgesunden männlichen Probanden im Alter von 20 bis 60 Jahren. Voraussetzung ist, daß alle Probanden eine durch manuelle Arbeit bedingte Hautstruktur der Handinnenflächen benutzen.

1.3. Testmengen

Eine definierte Menge Modellschmutz (bzw. Lack) und die Menge der Prüfsubstanz wird vor Beginn des Versuchs festgelegt.

1.4. Bewertungsschema

Die Bewertung erfolgt nach der Waschung nach einer 0-5 Punkte-Skala. Bewertet wird der Grad der Restverschmutzung auf der Handinnenfläche.

0     = sauber
1     = leichte Restverschmutzung
2     = mittlere Restverschmutzung
3     = starke Restverschmutzung
4     = sehr starke Restverschmutzung
5     = kein Reinigungseffekt

1.5. Durchführung

Morgens und nachmittags wird mit je einem Produkt folgende Waschung durchgeführt:
- 0,5 ml Modellschmutz (s. 1.1.1.) wird auf die Handinnenfläche aufgetragen und kurz verieben
- 3 Minuten trocknen lassen
- 0,5 ml des Testprodukts wird aufgetragen und kurz eingerieben
- ca. 1 ml Wasser wird zugefügt und 20 Sekunden gewaschen
- abspülen unter fließendem, kaltem Wasser
- Beurteilung der Reinigungswirkung nach der 5-Punkte-Skala

Die so erhaltenen Ergebnisse zeigt die Tabelle II:

Tabelle II

| Produkt/Proband | a | b | a | c | a | d | a | e | a | f |
|---|---|---|---|---|---|---|---|---|---|---|
| 1 | 2,5 | 4,5 | 2 | 3 | 2 | 3,5 | 1 | 1,5 | 1,5 | 2 |
| 2 | 3 | 4 | 3 | 4 | 2,5 | 4 | 2 | 2,5 | 2 | 2 |
| 3 | 2,5 | 4,5 | 3 | 2,5 | 3,5 | 4 | 2,5 | 2 | 2,5 | 2,5 |
| 4 | 3 | 4,5 | 3,5 | 2,5 | 2,5 | 4 | 2 | 2 | 0,5 | 2,5 |
| 5 | 3 | 3,5 | 2 | 3 | 2 | 3 | 2 | 3 | 1,5 | 2 |
| 6 | 3 | 4,5 | 3 | 3,5 | 4 | 3,5 | 1,5 | 2 | 1,5 | 1 |
| Ø | 2,8 | 4,3 | 2,8 | 3,1 | 2,8 | 3,7 | 1,8 | 2,2 | 1,6 | 2 |

Versuch II

In Versuch II wurde überprüft, ob der reinigungsverstärkte Effekt bei zusätzlicher Einarbeitung eines üblichen nichtlöslichen und nichtquellbaren Reibemittels (es wurde Polyethylenpulver gewählt) erhalten bleibt. Hierzu wurde in eine Standardrezeptur, die 7,5 % Polyethylenpulver enthält, eine bestimmte Menge wasserlösliches oder wasserquellbares Polymer gleicher Kornverteilung eingearbeitet und die Reinigungswirkung getestet.

Rezeptur III

15 Gew.-% Fettalkohol $C_{12}-C_{18}$ 5 EO (waschaktive
Substanz)

9,3 Gew.-% Dimethyladipat ⎫
36,3 Gew.-% Dimethylglutarat ⎬ (Lösemittel)
9,4 Gew.-% Dimethylsuccinat ⎭

5 Gew.-% iso-Octylstearat (Rückfettungsmittel)

2,3 Gew.-% pyrogene Kieselsäure ⎫
7 Gew.-% Ethylenglykoldistearat ⎬ (Verdickungs-
3,8 Gew.-% Celluloseacetobutyrat ⎭ mittel)

0,4 Gew.-% Parfüm

4,0 Gew.-% Polymerkomponenten a, c, d oder f
(gemäß Tabelle I kleiner als 45 μm

7,5 Gew.-% Polyethylenpulver kleiner als 300 μm

Die erhaltenen Ergebnisse zeigt Tabelle III

Tabelle III

| Produkt/ Proband | f | a | c | a | d | a |
|---|---|---|---|---|---|---|
| 1 | 2 | 1 | 0,5 | 1 | 3 | 0,5 |
| 2 | 1 | 1 | 1 | 1 | 3 | 1,5 |
| 3 | 1,5 | 1,5 | 1 | 1,5 | 2,5 | 1,5 |
| 4 | 1,5 | 1 | 1,5 | 2 | 3 | 1 |
| 5 | 1,5 | 1 | 1 | 1 | 2,5 | 2 |
| 6 | 0,5 | 1 | 1,5 | 2 | 2 | 1,5 |
| ∅ | 1,3 | 1,1 | 1,1 | 1,4 | 2,7 | 1,3 |

Versuch III

Versuch III zeigt, daß bei Verwendung eines erfindungsgemäßen Polymers bei der Reinigung eine wesentliche Menge Reinigungsprodukt eingespart werden kann, also das Schmutztragevermögen besser ist.
Rezeptur wie bei Versuch II; es wurde 0,5 ml des erfindunsgemäßen Produkts mit Polymerkomponente a gegen 1 ml des nichterfindungsgemäßen Produkts mit Polymerkomponente d zur Reinigung verwendet. Tabelle IV zeigt das erhaltene Ergebnis:

Tabelle IV:

Produkt/

| Proband | a (0,5 ml) | d (1,0 ml) |
|---------|-----------|-----------|
| 1 | 1 | 1,5 |
| 2 | 1 | 1,5 |
| 3 | 1 | 2 |
| 4 | 0,5 | 1 |
| 5 | 1 | 1,5 |
| 6 | 1 | 2 |
| ∅ | 0,9 | 1,6 |

Rezeptur IV

| | Rezeptur A | Rezeptur B | Rezeptur C | Rezeptur D | Rezeptur E |
|---|---|---|---|---|---|
| Fettalkohol $C_{12-18}$-5EO | 15 g | 15 g | 15 g | 15 g | 15 g |
| Dimethyladipat | 9,3 g | 9,3 g | 9,3 g | 9,3 g | 9,3 g |
| Dimethylglutarat | 36,3 g | 36,3 g | 36,3 g | 36,3 g | 36,3 g |
| Dimethylsuccinat | 9,4 g | 9,4 g | 9,4 g | 9,4 g | 9,4 g |
| Aerosil | 5,0 g | 5,0 g | 5,0 g | 5,0 g | 5,0 g |
| Polyethylenpulver kleiner 315 µm | 7,5 g | – | – | – | 7,5 g |
| Favor kleiner 315 µm | – | 7,5 g | – | – | – |
| Favor kleiner 45 µm | – | – | – | 4,0 g | 4,0 g |

Tabelle V

Handwaschtest:

Produktmenge 0,5 ml

| Proband | 0,5 ml Lack | | 0,3 ml Lack | | 0,7 ml Lack | |
|---|---|---|---|---|---|---|
| | A | B | C | D | A | E |
| 1 | 1,0 | 0,5 | 3,0 | 2,5 | 1,5 | 1,0 |
| 2 | 1,0 | 0,5 | 2,0 | 2,0 | 1,0 | 0,5 |
| 3 | 0,5 | 0 | 3,0 | 1,0 | 1,0 | 0,5 |
| 4 | 0 | 0,5 | 1,5 | 1,0 | 1,0 | 0,5 |
| 5 | 0,5 | 0,5 | 2,5 | 2,0 | 1,5 | 1,0 |
| X | 0,6 | 0,4 | 2,4 | 1,7 | 1,2 | 0,7 |

Versuch A gegen B: Reibemittel PE gegen Reibemittel
FAVOR

$\Longrightarrow$ kein wesentlicher Unterschied, bei dieser Korngröße kommt das Schmutztragevermögen kaum zum
Tragen.

Versuch C gegen D: ohne Reibemittel (FAVOR als
Schmutzträger)

```
==>   Schmutztragevermögen kommt deutlich heraus.


Versuch A gegen E:  Reibemittel PE gegen Reibemittel
              PE und Schmutzträger FAVOR


==>   Schmutztragevermögen wird auch in reibmittelhal-
      tigen Formulierungen deutlich.
```

Zusammenfassung:

Die durchgeführten Waschversuche zeigen deutlich die Überlegenheit von nicht wasserlöslichen, aber wasserquellbaren Polymeren (Favor SAB, Sunwet IM 1000, Aqua-Keep) gegenüber wasserlöslichen Polymeren (Versuch I). Versuch II zeigt, daß die Überlegenheit dieser Polymeren auch bei der Einarbeitung eines üblichen Reibemittels nicht verlorengeht. Das Ausmaß der Verbesserung durch ein erfindungsgemäßes Polymer zusammen mit einem üblichen Reibemittel wird in Versuch III deutlich. Mit der Hälfte des Reinigungsproduktes wird eine bessere Reinigung erzielt als mit der vollen Menge Produkt, das ein wasserlösliches Polymer enthält.

**Patentansprüche**

1. Wasserfreies Hautreinigungsmittel, enthaltend Tenside, organische Lösungsmittel und wasserunlösliche Reibmittel als wirksame Bestandteile, sowie gegebenenfalls Verdickungsmittel, Gerüststoffe, Rückfettungsmittel, Parfümstoffe, Konservierungsstoffe, Farbstoffe, Antioxidantien und/oder keratolytische Wirkstoffe, gekennzeichnet durch einen Gehalt an wasserunlöslichen, jedoch wasserquellbaren organischen Polymeren als Schmutzträger und gegbenenfalls als alleiniges Reibmittel oder zusammen mit weiteren an sich bekannten wasserunlöslichen Reibmitteln.

2. Hautreinigungsmittel nach Anspruch 1, gekennzeichnet durch einen Gehalt an natürlichen, gegebenenfalls modifizierten oder synthetischen organischen Polymeren.

3. Hautreinigungsmittel nach Anspruch 1 oder 2, gekennzeichnet durch, einen Gehalt an wasserquellbaren organischen Polymeren von 0,3 bis 55, vorzugsweise von 01, bis 17,6, insbesondere von 1 bis 12,5 Gew.-%, bezogen auf das Gesamtgewicht aus waschaktiver Substanz (Tensid), Lösemittel-und wasserquellbaren Polymer.

4. Hautreinigungsmittel nach einem der Ansprüche 1-3, gekennzeichnet durch einen Gehalt an wasserquellbaren Polymerisaten auf der Basis von Poly(meth)-acrylsäure und ihren Salzen, Pfropfmischpolymeren von Acrylderivaten mit Stärke- und Zellulose und deren Derivaten, Verseifungsprodukten von Poly(meth)acrylnitril, (Meth-)acrylamiden, Acrylestern und/oder Polyurethanen.

5. Hautreinigungsmittel nach Anspruch 4, gekennzeichnet durch einen Gehalt an wasserquellbaren Polymeren, die als Comonomere Acrylamidopropansulfonsäure, Vinylphosphonsäure, Vinylsulfonsäure, Dialkylaminoalkyl(meth) (-)acrylate, Dialkylaminoalkyl(meth-)acrylamide oder deren Quaternierungsformen enthalten.

6. Hautreinigungsmittel nach einem der Ansprüche 1 bis 5, gekennzeichnet durch einen Gehalt an alkoxylierten Fettalkoholen, alkoxylierten Fettsäuren, alkoxylierten Fettsäureglyceriden, alkoxylierten Alkylphenolen, Alkylsulfaten, Alkylethersulfaten, Sorbitan-Fettsäureestern und Fettsäureglyceriden als waschaktive Substanz.

7. Hautreinigungsmittel nach einem der Ansprüche 1 bis 6, gekennzeichnet durch einen Gehalt an Carbonsäurealkylestern, vorzugsweise Estern von ein- oder mehrbasischen Carbonsäuren mit 1 bis 10 Kohlenstoffatomen mit Alkoholen mit 1 bis 8, insbesondere 1 bis 4 Kohlenstoffatomen paraffinischen oder isoparaffinischen Kohlenwasserstoffen, Arylcarbonsäureestern oder Terpenen allein oder in Mischung untereinander als Lösemittel.

11

8. Hautreinigungssmittel nach einem der Ansprüche 1 bis 7, gekennzeichnet durch einen Gehalt an Zellulosederivaten, pyrogener Kieselsäure, modifizierten Bentoniten und/oder Wachsen als Verdickungsmittel und Gerüstbildner.

9. Hautreinigungsmittel nach einem der Ansprüche 1 bis 8, gekennzeichnet durch eine Korngröße der wasserquellbaren Polymeren von kleiner 200 µm, bevorzugt kleiner 150 µm, insbesondere kleiner 100 µm bei alleiniger Verwendung als Schmutzträger sowie von kleiner 1.000 µm, vorzugsweise kleiner 500 µm bei Verwendung als Schmutzfänger und Reibmittel.

10. Verwendung des Mittels nach Ansprüchen 1 bis 9 zur Reinigung der Haut, dadurch gekennzeichnet, daß man eine ausreichende Menge des Mittels auf die Haut aufträgt, dort verteilt und mit Wasser entfernt.

## Claims

1. Anhydrous skin cleansing agent comprising surfactants, organic solvents, and water-insoluble abrasives as active components, as well as optionally thickeners, builders, grease-restoring agents, perfumes, preserving agents, dyestuffs, antioxidants, and/or keratolytic agents, characterized by a content of water-insoluble, but water-swellable organic polymers as soil carrier, and optionally as the sole abrasive or in combination with other water-insoluble abrasives which are known per se.

2. Skin cleansing agent according to claim 1, characterized by a content of natural, optionally modified or synthetic organic polymers.

3. Skin cleansing agent according to claims 1 or 2, characterized by a content of water-swellable organic polymers of 0.3 to 55, preferably 0.1 to 17.6, particularly 1 to 12.5%-wt. relative to the total weight of detergent surfactant (surfactant), solvent, and water-swellable polymer.

4. Skin cleansing agent according to any one of claims 1 to 3, characterized by a content of water-swellable polymers on the basis of poly(meth-)acrylic acid and the salts thereof, graft copolymers of acrylic derivatives with starch and cellulose and their derivatives, saponification products of poly(meth-)acrylonitrile, (meth-)acrylamides, acrylic esters and/or polyurethanes.

5. Skin cleansing agent according to claim 4, characterized by a content of water-swellable polymers which comprise as comonomers acrylamidopropane sulfonic acid, vinylphosphonic acid, vinylsulfonic acid, dialkyl aminoalkyl(meth-)acrylates, dialkyl aminoalkyl(meth-) acrylamide, or the quaternization forms thereof.

6. Skin cleansing agent according to any one of claims 1 to 5 characterized by a content of alkoxylated fatty alcohols, alkoxylated fatty acids, alkoxylated fatty acid glycerides, alkoxylated alkylphenols, alkyl sulfates, alkyl ether sulfates, sorbitan fatty acid esters, and fatty acid glycerides as detergent surfactant.

7. Skin cleansing agent according to any one of claims 1 to 6, characterized by a content of carboxylic acid alkyl esters, preferably esters of mono- or polybasic carboxylic acids having 1 to 10 carbon atoms with alcohols having 1 to 8, particularly 1 to 4 carbon atoms, paraffinic or isoparaffinic hydrocarbons, aryl carboxylic acid esters or terpenes alone or in admixture among one another as solvent.

8. Skin cleansing agent according to any one of claims 1 of 7, characterized by a content of cellulose derivatives, fumed silica, modified bentonites, and/or waxes as thickeners and builders.

9. Skin cleansing agent according to any one of claims 1 to 8, characterized by a particle size of the water-swellable polymers of smaller than 200 µm, preferably smaller than 150 µm, particularly smaller than 100 µm, if exclusively used as soil carrier, as well as of smaller than 1,000 µm, preferably smaller than 500 µm, if used as soil carrier and abrasive.

10. The use of the agent as claimed in claims 1 to 9 for cleaning the skin, characterized in that a sufficient amount of the agent is applied to the skin, distributed thereon and removed with water.

**Revendications**

1. Composition de nettoyage de la peau, exempte d'eau, qui contient des agents tensioactifs ou surfactifs, des solvants organiques et des agents de gommage insolubles dans l'eau à titre de composants actifs, comme éventuellement aussi des agents épaississants, des adjuvants, des agents de regraissage, des parfums, des conservateurs, des colorants, des antioxydants et/ou des substances kératolytiques, caractérisée par une teneur en polymères organiques, insolubles dans l'eau, mais cependant suceptibles de gonfler à l'eau, à titre de véhicules de crasses ou saletés et éventuellement à titre d'agents de gommage uniques ou ensemble avec d'autres agents de gommage insolubles dans l'eau en soi connus.

2. Composition de nettoyage de la peau suivant la revendication 1, caractérisée par une teneur en polymères organiques naturels, éventuellement modifiés, ou synthétiques.

3. Composition de nettoyage de la peau suivant la revendication 1 ou 2, caractérisée par une teneur en polymères organiques susceptibles de gonfler à l'eau, de 0,3 à 55, de préférence de 0,1 à 17,6, plus particulièrement de 1 à 12,5, % en poids, par rapport au poids total de la substance détergente (tensioactif ou surfactif), du solvant et du polymère susceptible de gonfler à l'eau.

4. Composition de nettoyage de la peau suivant l'une des revendications 1-3, caractérisée par une teneur en polymères susceptibles de gonfler à l'eau à base de poly/acide(méth)acrylique/ et de ses sels, de copolymères de greffage, de dérivés acryliques avec l'amidon et la cellulose et leurs dérivés, les produits de saponification du poly(méth)acrylonitrile, de (méth)acrylamides, d'esters acryliques et/ou de polyuréthanes.

5. Composition de nettoyage de la peau suivant la revendication 4, caractérisée par une teneur en polymères susceptibles de gonfler à l'eau, qui contiennent, à titre de comonomères, de l'acide acrylamidopropanesulfonique, de l'acide vinylphosphonique, de l'acide vinylsulfonique, des (méth)acrylates de dialkylaminoalkyle, des dialkylaminoalkyl(méth)acrylamides, ou leurs formes quaternisées.

6. Composition de nettoyage de la peau suivant l'une quelconque des revendications 1 à 5, caractérisée par une teneur en alcools gras alcoxylés, en acides gras alcoxylés, en glycérides d'acides gras alcoxylés, en alkylphénols alcoxylés, en alkylsulfates, en alkyléthersulfates, en esters d'acides gras du sorbitan et en glycérides d'acides gras, à titre de substances détergentes.

7. Composition de nettoyage de la peau suivant l'une quelconque des revendications 1 à 6, caractérisée par une teneur en carboxylates d'alkylesters, de préférence des esters d'acides carboxyliques mono- ou polybasiques, qui comportent de 1 à 10 atomes de carbone, avec des alcools qui possèdent de 1 à 8, plus particulièrement 1 à 4, atomes de carbone, en hydrocarbures paraffiniques ou isoparaffiniques, en esters d'acides arylcarboxyliques, ou en terpènes, seuls ou en mélanges les uns aux autres, à titre de solvants.

8. Composition de nettoyage de la peau suivant l'une quelconque des revendications 1 à 7, caractérisée par une teneur en dérivés de la cellulose, en silice colloîdale ou pyrogénée, en bentonites modifiées et/ou en cires, à titre d'agents épaississants et d'adjuvants de nettoyage.

9. Composition de nettoyage de la peau suivant l'une quelconque des revendications 1 à 8, caractérisée par un calibre des grains des polymères susceptibles de gonfler à l'eau inférieur à 200 μm, de préférence inférieur à 150 μm, plus particulièrement inférieur à 100 μm, lorsqu'on les utilise uniquement à titre de véhicules de crasses ou saletés, comme aussi inférieur à 1000 μm, de préférence inférieur à 500 μm, lors de leur emploi à titre d'agents de gommage et de captation des crasses ou saletés.

10. Utilisation de la composition suivant l'une quelconque des revendications 1 à 9 en vue du nettoyage de la peau, caractérisée en ce que l'on applique une proportion suffisante de la composition sur la peau, on l'y répartit et on l'en enlève avec de l'eau.